# FASCICULE DE BREVET EUROPEEN

(11) **EP 0 723 955 B1**
(45) Date de publication et mention de la délivrance du brevet: **06.10.1999**
(21) Numéro de dépôt: 96400121.8
(22) Date de dépôt: 18.01.1996
(51) Int. Cl.: C07C 267/00

(54) **Procédé de synthèse de carbodiimides substitués**
Verfahren zur Herstellung substituierter Carbodiimide
Process for the preparation of substituted carbodiimides

(30) Priorité: 24.01.1995 FR 9500748
(43) Date de publication de la demande: 31.07.1996
(73) Titulaire: SOCIETE NATIONALE DES POUDRES ET EXPLOSIFS, F-75181 Paris Cédex 04 (FR)
(72) Inventeur: Hussenet, Patricia, F-91200 Athis Mons (FR); Le Goff, Philippe, F-67000 Strasbourg (FR); Sennyey, Gérard, F-91190 Saint Aubin (FR)
(74) Mandataire: Pech, Bernard

(56) Documents cités:
- FR-A- 1 275 307
- TETRAHEDRON, vol. 37, 1981, OXFORD GB, pages 233-284, XP002001915 M. MIKOLAJCZYK: "Recent developments in the carbodiimide chemistry"
- CHEMISCHE BERICHTE, vol. 91, 1958, WEINHEIM DE, page 1992-95 XP002001916 J. MACHOLDT-ERDNISS: "Eine einfache Darstellung aromatischer Guanidine"
- ANGEWANDTE CHEMIE, vol. 72, no. 22, 1960, XP002001917 EILINGSFELD ET AL.:
- JOURNAL OF ORGANIC CHEMISTRY, vol. 29, 1964, EASTON US, pages 2401-2404, XP002001918 H. ULRICH ET AL.: "Syntheses and some reactions of allophanoyl chlorides"

## Description

La présente invention est relative à un nouveau procédé de synthèse de carbodiimides disubstitués à partir d'urées N,N'-disubstituées. Les carbodiimides disubstitués, en particulier le N,N'-dicyclohexylcarbodiimide (DCC), sont notamment utiles comme intermédiaires de synthèse en chimie organique, par exemple en chimie pharmaceutique comme agents de couplage, notamment en synthèse peptidique. Lors des étapes de couplage, les carbodiimides disubstitués sont transformés en urées N,N'-disubstituées correspondantes. Le procédé selon l'invention est donc particulièrement intéressant puisqu'il permet le recyclage d'un sous-produit en matière première.

Il est connu d'obtenir des carbodiimides disubstitués par réaction, en milieu solvant organique, d'un agent d'halogénation avec une urée N,N'-disubstituée.

PALOMO et MESTRES obtiennent, d'après Chemical Abstracts 95 (9) : 80095j, des carbodiimides disubstitués par réaction d'urées N,N'-disubstituées avec (C₆H₅)₃PBr₂ en présence de triéthylamine et en milieu CH₂Cl₂. Le rendement est de 90%.

HARUTA, SUEMATSU et NAKAOKA obtiennent, d'après Chemical Abstracts 105 (9) : 78567w et Chemical Abstracts 104 (9) : 68491g, le DCC par réaction de la N,N'-dicyclohexylurée (DCU) avec POCl₃ en présence de pyridine. Les rendements sont de 79-80%.

Ces procédés présentent l'inconvénient d'induire des rejets et effluents phosphorés, ce qui est très pénalisant au stade industriel du fait de la nécessité de traitements de ces rejets et effluents.

De plus, les rendements ne sont pas très élevés (80 à 90% environ) et le carbodiimide brut obtenu après évaporation du solvant doit être purifié par distillation.

Pour s'affranchir des rejets et effluents phosphorés, il est connu d'utiliser le phosgène comme agent de chloration de l'urée.

Le brevet japonais JP 54076559 décrit l'obtention du DCC, en 2 étapes, par phosgénation de la DCU.

Dans une première étape, les auteurs obtiennent et isolent du milieu réactionnel, le chlorure de N,N'-dicyclohexylchloroformamidinium (DCFC) obtenu par phosgénation de la DCU en milieu dialkyléther. Cet intermédiaire DCFC est ensuite traité, dans une seconde étape, par une solution aqueuse de soude en présence de dichlorométhane, ce qui permet d'obtenir le DCC. Le rendement global d'obtention du DCC à partir de la DCU est compris entre 82% et 88%.

Ce procédé en deux étapes est long, coûteux, et de rendement inférieur à 90%. Il requiert l'isolement, par filtration et séchage, du composé intermédiaire DCFC, particulièrement sensible à l'hydrolyse, donc délicat à manipuler. Il nécessite deux réacteurs, deux solvants différents et, si les effluents et rejets ne contiennent pas de dérivés phosphorés, leur quantité est de ce fait importante.

Les procédés précités ne sont donc pas satisfaisants, notamment à l'échelle industrielle, et l'homme du métier recherche un procédé simple de mise en oeuvre, bon marché, procurant des rendements plus élevés que ceux jusqu'alors obtenus, et qui soit écologique, c'est à dire induisant des effluents et rejets en faible quantité et dépourvus de composés toxiques.

La présente invention propose un tel procédé, et donc une solution aux problèmes précités.

Elle a pour objet un procédé simple et peu onéreux d'obtention d'un carbodiimide disubstitué comprenant une première étape de réaction, en milieu solvant organique, du phosgène avec une urée N,N'-disubstituée.

Elle est caractérisée en ce que, après cette première étape réactionnelle de phosgénation de l'urée N,N'-disubstituée, sans isoler au préalable de composé intermédiaire, et notamment le chlorure de chloroformamidinium formé au cours de cette première étape, on ajoute de l'ammoniac dans le milieu réactionnel.

Cette façon d'opérer est particulièrement intéressante au stade industriel du fait de sa simplicité et de son faible coût. Le procédé est mis en oeuvre dans un seul réacteur, ne requiert qu'un seul solvant et l'ammoniac est un réactif bon marché. Les effluents et rejets sont peu ou pas toxiques et en faible quantité.

Ce procédé permet également, de façon inattendue, d'obtenir, d'une part d'excellents rendements, supérieurs à 95%, même parfois à 98%, et d'autre part un carbodiimide brut de synthèse très pur, 99% environ, ne nécessitant pas de purification particulière.

Un tel résultat est particulièrement surprenant pour plusieurs raisons.

Tout d'abord, comme le montrent les exemples comparatifs mentionnés dans la présente demande, ce résultat n'est pas obtenu lorsqu'on utilise, au lieu de l'ammoniac, la pyridine ou une amine tertiaire, bases azotées jusqu'alors utilisées en association avec l'agent chlorurant, comme selon l'état de la technique précité. Ce résultat ne provient donc pas de la seule simplification du procédé (un seul réacteur, un seul solvant, pas d'isolement du dérivé intermédiaire), mais d'une synergie entre cette simplification et le choix d'une base particulière, l'ammoniac.

Par ailleurs, ce résultat est d'autant plus surprenant qu'il était connu de l'homme du métier que l'ammoniac, les amines primaires et les amines secondaires réagissent avec les carbodiimides pour former les guanidines correspondantes. On peut se référer par exemple à MIKOLAJCZYK et KIELBASINSKI, Tétrahedron, vol 37, Recent developments in the carbodiimide chemistry, page 245, ainsi qu'à J. MACHOLDT-ERDNISS, Chemische Berichte, vol. 91, 1958, Eine einfache Darstellung aromatischer Guanidine, page 1992.

C'est d'ailleurs très certainement la raison pour laquelle l'homme du métier n'utilisait jusqu'alors, pour obtenir des carbodiimides à partir d'urées, que la pyridine ou les amines tertiaires comme bases azotées.

De plus, il était également connu que les amines primaires réagissent avec l'intermédiaire chlorure de chloroformamidinium pour former des guanidines. On peut se référer par exemple à EILINGSFELD, SEEFELDER et WEI-DINGER, Angew. Chem. 72, 1960, N° 22, Amidchloride und Carbamidchloride, page 845.

L'homme du métier, pour toutes ces raisons, était donc très fortement dissuadé d'utiliser, de façon générale, l'ammoniac pour transformer un chlorure de chloroformamidinium en carbodiimide correspondant. Il l'était d'autant plus que son but était notamment d'améliorer nettement les rendements jusqu'alors obtenus selon les procédés connus.

La Demanderesse n'est pas en mesure de proposer d'explication pouvant justifier, même à posteriori, les résultats constatés.

Le procédé selon la présente invention est mis en oeuvre en milieu solvant organique. Le terme "solvant" ne doit pas être entendu dans un sens limitatif. Il faut comprendre qu'on utilise un milieu organique non réactif dans les conditions opératoires, appelé couramment "solvant" organique. Selon la nature de l'urée de départ et du "solvant" organique, l'urée peut être en suspension et/ou en solution dans le solvant organique.

Pour mettre en oeuvre la présente invention, chaque atome d'azote de l'urée doit porter un atome d'hydrogène, c'est à dire que la terminologie "N,N'-disubstituée" qualifiant l'urée doit être comprise dans un sens limitatif, excluant que l'urée soit N,N'-tri ou tétrasubstituée.

Selon la présente invention, l'urée N,N'-disubstituée répond de préférence à la formule générale (I) suivante : 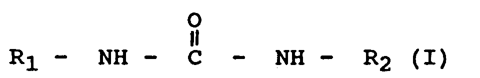 dans laquelle R₁ et R₂, identiques ou différents, de préférence identiques, représentent :
- un groupement phényle, non substitué, ou substitué, de préférence par un atome d'halogène ou une chaîne alkyle, C₁ - C₄ par exemple,
- une chaîne alkyle linéaire ou ramifiée, de préférence C₁ - C₈, mieux encore C₁ - C₄, non substituée, ou substituée, par exemple par un atome d'halogène,
- une chaîne cycloalkyle, de préférence C₅ - C₆, non substituée ou substituée par un atome d'halogène ou une chaîne alkyle, C₁ - C₄ par exemple.

Comme exemples d'urées particulièrement préférées, on peut citer la N,N'-diisopropylurée et la N,N'-dicyclohexylurée.

Lorsque l'urée répond à la formule générale (I), le procédé selon l'invention permet d'obtenir un carbodiimide disubstitué de formule générale (II), selon le schéma réactionnel suivant, par l'intermédiaire d'un chlorure de chloroformamidinium de formule générale (III) :

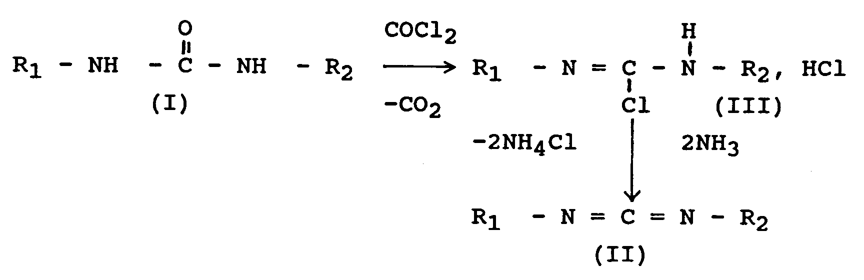

Selon l'invention, le carbodiimide disubstitué obtenu peut être isolé du milieu réactionnel par tout moyen connu de l'homme du métier.

On peut, par exemple, selon une première variante, filtrer le milieu réactionnel (le résidu recueilli sur le filtre est essentiellement du chlorure d'ammonium), recueillir le filtrat, puis chasser le solvant, par évaporation sous pression réduite de préférence.

On constate que cette variante permet d'obtenir un carbodiimide brut de synthèse très pur, ne nécessitant pas de purification particulière, selon un procédé mis en oeuvre sans aucune utilisation d'eau.

On peut aussi, selon une seconde variante, effectuer une extraction aqueuse du milieu réactionnel, qui a pour but essentiel d'extraire le chlorure d'ammonium formé, suivie d'une évaporation du solvant organique, sous pression réduite par exemple.

On obtient également, selon cette variante, un carbodiimide brut de synthèse très pur ne nécessitant pas de purification particulière, distillation par exemple.

Selon l'invention, la première étape de phosgénation de l'urée N,N'-disubstituée pour former le chlorure de chloroformamidinium comprend une première opération d'introduction du phosgène dans une suspension ou une solution de l'urée dans le solvant organique, suivie d'une seconde opération de dégazage du milieu réactionnel, par un gaz inerte, azote ou argon par exemple, ou sous pression réduite. Lorsque l'urée est faiblement soluble dans le solvant, l'urée peut être utilisée en suspension dans la solution saturée.

De façon préférée, on opère en présence d'un léger excès de phosgène, c'est à dire plus précisément que le rapport molaire entre le phosgène et l'urée est compris entre 1,1 et 1,5.

Bien que tout solvant organique puisse convenir pour mettre en oeuvre le procédé selon l'invention, notamment les solvants chlorés comme CHCl₃ ou CH₂Cl₂ et le tétrahydrofuranne (THF), on préfère utiliser un dérivé éther, notamment un dialkyléther.

Comme exemples de dialkyléthers préférés, on peut citer ceux pour lesquels les chaînes alkyles comportent de 1 à 5 atomes de carbone, comme par exemple le diéthyléther, le diisopropyléther, le diisobutyléther, le méthyl butyléther, le méthyl isopropyléther et le méthyl tertiobutyléther. Ce dernier est particulièrement préféré.

De façon préférée, d'une part la durée d'introduction du phosgène est supérieure à 3h, et d'autre part la température du milieu réactionnel est comprise entre environ 15°C et environ 25°C.

Par ailleurs, pendant l'opération de dégazage, la température du milieu réactionnel est de préférence comprise entre Eb - 3°C et Eb - 10°C, Eb étant la température d'ébullition du solvant organique, en degrés Celcius, à la pression atmosphérique normale (101325 Pa, soit 760mm Hg), sans toutefois dépasser 70°C.

Selon l'invention, après l'opération précitée de dégazage, on introduit de l'ammoniac, de préférence à l'état gazeux, dans le milieu réactionnel, sans isoler au préalable de composé intermédiaire, et sans aucun traitement du milieu réactionnel autre qu'un éventuel traitement thermique pour abaisser sa température.

Pendant l'introduction de l'ammoniac, la température du milieu réactionnel est de préférence comprise entre - 5°C et 10°C, mieux encore entre 0°C et 8°C.

De façon préférée, on utilise un léger excès d'ammoniac par rapport à la stoechiométrie, c'est à dire que le rapport molaire entre l'ammoniac et l'urée N,N'-disubstituée est compris entre 2 et 3.

Les exemples non limitatifs suivants illustrent l'invention et les avantages qu'elle procure.

### Exemple 1 Synthèse de DCC selon l'invention avec élimination du chlorure d'ammonium par filtration

Dans un réacteur double enveloppe de 21 équipé d'une agitation mécanique, d'une sonde thermométrique, d'une canne d'introduction de gaz et d'un réfrigérant à carboglace surmonté d'un système d'échappement de gaz, on introduit, à température ambiante et sous balayage d'azote, 740g (1l) de méthyl, tertiobutyléther (TBME) et 228,3g de DCU. On obtient une suspension dans laquelle on introduit, de façon continue, sous vive agitation, 131g de phosgène, tout en maintenant la température du milieu réactionnel entre 15°C et 20°C. La durée de l'introduction du phosgène est de 3,5h.

Après l'introduction du phosgène, on élève la température du milieu réactionnel jusqu'à 50°C, puis on dégaze le milieu pendant 1h par barbotage d'azote.

On abaisse ensuite la température du milieu réactionnel jusqu'à 2°C.

On introduit alors dans le milieu, par bullage, 43g d'ammoniac gazeux, tout en maintenant sa température inférieure à 6°C.

Après l'introduction de l'ammoniac, puis après avoir laissé remonter la température du milieu jusqu'à la température ambiante (20°C environ), on filtre le milieu pour récupérer le filtrat. On lave le résidu de filtration (essentiellement le chlorure d'ammonium formé au cours de la réaction) sur le filtre avec 3 fois 300ml de TBME, puis on regroupe les filtrats.

On évapore alors le solvant, sous pression réduite (15mm Hg, soit environ 2000 Pa), ce qui permet d'obtenir 207g d'une huile légèrement colorée qui fige à la température ambiante. Le spectre IR de ce produit est conforme au spectre de référence du DCC. Sa pureté, déterminée par chromatographie en phase gazeuse, est de 98,5%. Le rendement, par rapport à la DCU de départ, est de 98,7%.

### Exemple 2 Synthèse de DCC selon l'invention avec élimination du chlorure d'ammonium par extraction aqueuse

Pour cet exemple 2, jusqu'à la fin de l'introduction de l'ammoniac, on opère exactement comme selon l'exemple 1, sauf qu'on introduit 122g de phosgène pendant 3,25h au lieu de 131g pendant 3,5h, et que l'on introduit 45g d'ammoniac au lieu de 43g.

Après l'introduction de l'ammoniac et après avoir laissé remonter la température du milieu jusqu'à la température ambiante, on ajoute 400g d'eau puis on agite durant 1h.

Après décantation, on récupère la phase organique, puis on repète 3 fois de plus, avec à chaque fois 200g d'eau et durant 20min, cette opération d'extraction aqueuse du chlorure d'ammonium formé.

Après la dernière décantation, on sèche la phase organique sur sulfate de magnésium, puis on évapore le solvant sous pression réduite (2000 Pa environ), ce qui permet d'obtenir 200g d'une huile légèrement colorée qui fige à la température ambiante. Les spectres IR et RMN 1H et 13C sont conformes aux spectres de référence du DCC. La pureté du DCC obtenu, déterminée par chromatographie en phase gazeuse, est de 99,5%.

Le rendement, par rapport à la DCU de départ, est de 95,3%.

### Exemples comparatifs A et B

Ces 2 exemples comparatifs ne font pas partie de l'invention. Ils ont été réalisés dans le seul but de montrer que le choix de l'ammoniac, comme base pour transformer le chlorure de chloroformamidinium en carbodiimide, n'est pas arbitraire, mais nécessaire pour procurer l'effet technique constaté et les résultats qui en découlent (rendements très élevés).

Pour ces exemples A et B, on a opéré de façon rigoureusement identique à celle décrite pour l'exemple 2, sauf que l'on a utilisé, au lieu de l'ammoniac, pour l'exemple A 200g de pyridine ajoutés goutte à goutte, et pour l'exemple B 250g de triéthylamine ajoutés goutte à goutte.

Le rendement en DCC obtenu, par rapport à la DCU de départ, est de 66% pour l'exemple A et de 80% pour l'exemple B.

On constate donc que ces rendements sont très nettement inférieurs à celui obtenu pour l'exemple 2, et qu'ils sont voisins, même un peu inférieurs, de ceux obtenus selon les procédés d'obtention du DCC à partir de DCU précités dans l'état de la technique.

## Revendications

1. Procédé de synthèse d'un carbodiimide disubstitué comprenant une première étape de phosgénation, en milieu solvant organique, d'une urée N,N'-disubstituée, caractérisé en ce que, après cette première étape réactionnelle, sans isoler au préalable de composé intermédiaire, on ajoute de l'ammoniac dans le milieu réactionnel.

2. Procédé de synthèse selon la revendication 1, caractérisé en ce que l'urée N,N'-disubstituée répond à la formule générale (I) : 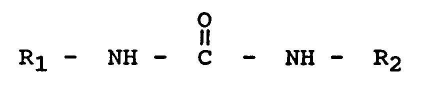 dans laquelle R₁ et R₂, identiques ou différents, représentent :
- un groupement phényle, substitué ou non substitué,
- une chaîne alkyle C₁ - C₈, substituée ou non substituée,
- une chaîne cycloalkyle C₅ - C₆, substituée ou non substituée.

3. Procédé de synthèse selon la revendication 1, caractérisé en ce que l'urée N,N'-disubstituée est la N,N'-dicyclohexylurée.

4. Procédé de synthèse selon la revendication 1, caractérisé en ce que, après l'addition d'ammoniac, on isole le carbodiimide formé par filtration ou extraction aqueuse du milieu réactionnel, suivie d'une évaporation du solvant organique.

5. Procédé de synthèse selon la revendication 1, caractérisé en ce que la première étape de phosgénation de l'urée N,N'-disubstituée comprend une première opération d'introduction du phosgène dans une suspension ou une solution de l'urée dans le solvant organique suivie d'une seconde opération de dégazage du milieu réactionnel, par un gaz inerte ou sous pression réduite.

6. Procédé de synthèse selon la revendication 1, caractérisé en ce que le solvant organique est un dialkyléther.

7. Procédé de synthèse selon la revendication 5, caractérisé en ce que la durée de l'introduction du phosgène est supérieure à 3h, et en ce que, pendant l'introduction du phosgène, la température du milieu réactionnel est comprise entre 15°C et 25°C.

8. Procédé de synthèse selon la revendication 5, caractérisé en ce que, pendant l'opération de dégazage, la température du milieu réactionnel est comprise entre Eb - 3°C et Eb - 10°C, Eb étant la température d'ébullition du solvant organique, en °C, à la pression normale, sans toutefois dépasser 70°C.

9. Procédé de synthèse selon la revendication 1, caractérisé en ce que, pendant l'addition d'ammoniac, la température du milieu réactionnel est comprise entre - 5°C et 10°C.

10. Procédé de synthèse selon la revendication 1, caractérisé en ce que le rapport molaire entre le phosgène et l'urée N,N'-disubstituée est compris entre 1,1 et 1,5 et en ce que le rapport molaire entre l'ammoniac et l'urée N,N'-disubstituée est compris entre 2 et 3.

## Claims

1. Method for synthesizing a disubstituted carbodiimide comprising a first phosgenation step, in an organic solvent medium, from an N,N'-disubstituted urea, characterized in that, after this first step of the reaction, ammonia is added to the reaction medium without previously isolating the intermediate compound.

2. Synthetic method according to claim 1, characterized in that the N,N'-disubstituted urea corresponds to the general formula (I): 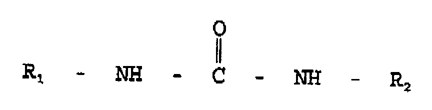 in which R₁, and R₂, identical or different, represent:
- a substituted or unsubstituted phenyl group,
- a substituted or unsubstituted C₁ - C₈ alkyl chain,
- a substituted or unsubstituted C₅ - C₆ cycloalkyl chain.

3. Synthetic method according to claim 1, characterized in that the N,N'-disubstituted urea is N,N'-dicyclohexylurea.

4. Synthetic method according to claim 1, characterized in that, after adding ammonia, the carbodiimide formed is isolated by filtration or aqueous extraction of the reaction medium, followed by evaporation of the organic solvent.

5. Synthetic method according to claim 1, characterized in that the first step of phosgenating the N,N'-disubstituted urea comprises a first operation of introducing phosgene into a suspension or a solution of urea in the organic solvent followed by a second operation of degassing the reaction medium with an inert gas or under reduced pressure.

6. Synthetic method according to claim 1, characterized in that the organic solvent is a dialkylether.

7. Synthetic method according to claim 5, characterized in that the phosgene introduction lasts more than 3 hours, and in that, during the introduction of phosgene, the temperature of the reaction medium lies between 15°C and 25°C.

8. Synthetic method according to claim 5, characterized in that, during the degassing operation, the temperature of the reaction medium lies between Eb-3°C and Eb-10°C, Eb being the boiling point of the organic solvent, in °C, at normal pressure, without however exceeding 70°C.

9. Synthetic method according to claim 1, characterized in that, during the addition of ammonia, the temperature of the reaction medium lies between -5°C and 10°C.

10. Synthetic method according to claim 1, characterized in that the molar ratio between phosgene and the N,N'-disubstituted urea lies between 1.1 and 1.5 and in that the molar ratio between ammonia and the N,N'-disubstituted urea lies between 2 and 3.

## Patentansprüche

1. Verfahren zur Herstellung eines disubstituierten Carbodiimids, das einen ersten Schritt, in dem ein N,N'-disubstituierter Harnstoff in einem organischen Lösungsmittelmedium mit Phosgen umgesetzt wird, umfaßt, dadurch gekennzeichnet, daß nach diesem ersten Reaktionsschritt und ohne vorherige Isolierung von Zwischenprodukten Ammoniak zu dem Reaktionsmedium gegeben wird.

2. Herstellungsverfahren nach Anspruch 1, dadurch gekennzeichnet, daß der N,N'-disubstituierte Harnstoff die allgemeine Formel (I) 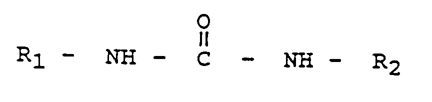 aufweist, in der R₁ und R₂, die gleich oder verschieden sind, bedeuten:
- eine Phenylgruppe, die substituiert oder nicht substituiert ist,
- eine C₁-C₈-Alkylgruppe, die substituiert oder nicht substituiert ist,
- eine C₅-C₆-Cycloalkylgruppe, die substituiert oder nicht substituiert ist.

3. Herstellungsverfahren nach Anspruch 1, dadurch gekennzeichnet, daß es sich bei dem N,N'-disubstituierten Harnstoff um N,N'-Dicyclohexylharnstoff handelt.

4. Herstellungsverfahren nach Anspruch 1, dadurch gekennzeichnet, daß nach der Zugabe des Ammoniaks das gebildete Carbodiimid durch Filtration oder wäßrige Extraktion des Reaktionsmediums isoliert wird, worauf eine Verdampfung des organischen Lösnungsmittels folgt.

5. Herstellungsverfahren nach Anspruch 1, dadurch gekennzeichnet, daß der erste Verfahrensschritt, in dem der N,N'-disubstituierte Harnstoff mit Phosgen umgesetzt wird, einen ersten Arbeitsgang, in dem Phosgen zu einer Suspension oder eine Lösung des Harnstoffs in dem organischen Lösungsmittel gegeben wird, und einen darauf folgenden zweiten Arbeitsgang, in dem das Reaktionsmedium mit einem Inertgas oder unter vermindertem Druck entgast wird, umfaßt.

6. Herstellungsverfahren nach Anspruch 1, dadurch gekennzeichnet, daß das organische Lösungsmittel aus einem Dialkylether besteht.

7. Herstellungsverfahren nach Anspruch 5, dadurch gekennzeichnet, daß die Zugabe des Phosgens länger als 3 h dauert und daß die Temperatur des Reaktionsmediums während der Zugabe des Phosgens im Bereich von 15 bis 25 °c liegt.

8. Herstellungsverfahren nach Anspruch 5, dadurch gekennzeichnet, daß die Temperatur des Reaktionsmediums während des Arbeitsgangs, in dem die Entgasung durchgeführt wird, im Bereich von Sdp. -3 °C bis Sdp. - 10 °C, wobei Sdp. der in °C angegebene Siedepunkt des organischen Lösungsmittels bei Normaldruck ist, liegt, ohne jedoch 70 °C zu übersteigen.

9. Herstellungsverfahren nach Anspruch 1, dadurch gekennzeichnet, daß die Temperatur des Reaktionsmediums während der Zugabe des Ammoniaks im Bereich von -5 bis 10 °C liegt.

10. Herstellungsverfahren nach Anspruch 1, dadurch gekennzeichnet, daß das Molverhältnis Phosgen zu N,N'-disubstituiertem Harnstoff im Bereich von 1,1 bis 1,5 und das Molverhältnis von Ammoniak zu N,N'-disubstituiertem Harnstoff im Bereich von 2 bis 3 liegt.
